# EUROPÄISCHE PATENTANMELDUNG

(11) **EP 3 192 433 A1**
(43) Veröffentlichungstag der Anmeldung: **19.07.2017**
(21) Anmeldenummer: 17000058.2
(22) Anmeldetag: 13.01.2017
(51) Int. Cl.: A61B 3/11, G02C 13/00

(54) **MOBILES VIDEOZENTRIERSYSTEM ZUR BESTIMMUNG VON ZENTIERDATEN FÜR BRILLENGLÄSER**

(30) Priorität: 14.01.2016 DE 202016000170 U
(71) Anmelder: Ollendorf, Hans-Joachim, 39517 Brunkau (DE)
(72) Erfinder: Ollendorf, Hans-Joachim, 39517 Brunkau (DE)
(74) Vertreter: Patentanwälte Schuster, Müller & Partner mbB

(57) **Zusammenfassung**

Die Erfindung geht aus von einem mobilen Videozentriersystem zur Ermittlung von Zentrierdaten für Brillengläser, bestehend aus einem Tablet-PC (1), der ein Display, einen Lagesensor und an einem der beiden vertikalen Randbereiche der dem Display abgewandten Seite eine Bilderfassungseinrichtung (2) aufweist, wobei diese Seite des Tablet-PC's (1) mit einem vom Objektiv dieser Bilderfassungseinrichtung (2) zumindest bis zur Mitte des Tablet-PC's (1) reichenden optischen Aufsatz (3) versehen ist, der eine Austrittsöffnung (4) aufweist, die sich in der Ebene der vertikalen Mitte dieser Seite des Tablet-PC's (1) befindet.

Erfindungsgemäß weist der Tablet-PC (1) eine hochauflösende Bilderfassungseinrichtung (2) auf und in dem optischen Aufsatz (3) ist ein Periskop mit Umlenkspiegeln (5, 6) angeordnet.

Ein solches mobiles Videozentriersystem hat den Vorteil, dass es aufgrund seiner hochauflösenden Bilderfassungseinrichtung ohne ein in den Strahlengang des Periskops eingebautes Fernrohr auskommt, wodurch das Videozentriersystem auch leichter ist.

## Beschreibung

### Stand der Technik

Die Erfindung geht aus von einem mobilen Videozentriersystem zur Ermittlung von Zentrierdaten für Brillengläser, wie z. B Hornhautscheitelabstand, Pupillendistanz, Einschleifhöhe, Vorneigungs- und Fassungsscheibenwinkel u. a.

Um Zentrierdaten für Brillengläser zu bestimmen, ist es bekannt, mittels einer Kamera ein frontales digitales Bild von dem Gesicht des Brillenträgers mit aufgesetztem Brillengestell anzufertigen und die für die Bestimmung der Zentrierdaten relevanten Positionen und Abstände anhand dieses Bildes auszuwerten. Hierzu ist bereits ein für den Optiker leicht zu handhabendes mobiles Videozentriersystem bekannt, das aus einem Tablet-PC besteht, der ein Display, einen Lagesensor und an einem der beiden vertikalen Randbereiche der dem Display abgewandten Seite eine Bilderfassungseinrichtung aufweist. Diese, also einem Probanden zugewandte Seite des Tablet-PC's ist mit einem vom Objektiv dieser Bilderfassungseinrichtung zumindest bis zur Mitte des Tablet-PC's (1) reichenden optischen Aufsatz versehen, der eine Austrittsöffnung aufweist, die sich in der Ebene der vertikalen Mitte dieser Seite des Tablet-PC's befindet. In dem optischen Aufsatz ist ein Periskop und zwischen dessen Umlenkspiegeln ein die Brennweite des Objektivs der Bilderfassungseinrichtung vergrößerndes Linsensystem angeordnet (DE 20 2012 000 167 U1).

Der Nachteil dieses mobilen Videozentriersystems besteht darin, dass es im Vergleich zu derzeit zur Verfügung stehenden kleineren mobilen Kommunikationsgeräten immer noch verhältnismäßig groß und somit unhandlich ist.

### Die Erfindung und ihre Vorteile

Das erfindungsgemäße mobile Videozentriersystem mit den Merkmalen des Schutzanspruchs 1 hat demgegenüber den Vorteil, dass es aufgrund seiner hochauflösenden Bilderfassungseinrichtung ohne ein in den Strahlengang des Periskops eingebautes Fernrohr auskommt, wodurch das Videozentriersystem auch leichter ist. Unter einer hochauflösenden Bilderfassungseinrichtung sind Kameras mit einer Auflösung von mindestens 8 Megapixel zu verstehen. Außerdem ist es in seiner Herstellung preisgünstiger. Um das Bild des Probanden in einer größeren Entfernung machen zu können, wird die elektronische Vergrößerung, der sog. Zoom, der hochauflösenden Kamera des Tablet-PC's verwendet. Der zentrierte Bildeintritt in das mobile Videozentriersystem ist erforderlich, um während der Aufnahme eine vom Optiker über die Bilderfassungseinrichtung zum Probanden zielende Gerade, eine sog. Fixierlinie, zu gewährleisten.

Zum Zweck der Gewinnung zuverlässiger Daten aus den Aufnahmen des Gesichts des Probanden ist es bei der Videozentrierung erforderlich, die Konvergenz durch eine Korrekturrechnung auszugleichen. Dies ist bei zu kurzer Aufnahmeentfernung zum Probanden nicht mehr möglich. Bei einer größeren Aufnahmeentfernung kann durch die elektronische Vergrößerung heutiger moderner hochauflösender Kameras von Tablet-PC's der Nachteil des Verlustes von für die Videozentrierung wesentlichen Details vermieden werden.

Nach einer vorteilhaften Ausgestaltung der Erfindung wird ein Tablet-PC verwendet, dessen Display nicht größer als 8 Zoll ist. Somit ist das mobile Videozentriersystem schon alleine von seinen Abmessungen her deutlich kleiner als eines, das einen Tablet-PC mit einem 9,7-Zoll-Monitor verwendet. Damit steht dem Optiker ein kompaktes und dadurch auch leichter als das herkömmliche mobile Videozentriersystem zu handhabendes Gerät zur Verfügung. Außerdem ist bei solchen verhältnismäßig kleinen Tablet-PC's der Abstand zwischen Bilderfassungseinrichtung und der Mitte seines optischen Aufsatzes, in der sich die Austrittsöffnung befindet, verhältnismäßig klein, sodass sich die für die zur Ermittlung von Zentrierdaten für Brillengläser erforderlichen Werte auch ohne ein in den Strahlengang eingebautes Linsensystem noch ausreichend genau aus dem auf dem Display erscheinenden Bild des Gesichts des Probanden ermitteln lassen.

Nach einer ebenfalls vorteilhaften Ausgestaltung der Erfindung ist der optische Aufsatz parallel zum oberen Rand des Tablet-PC's angeordnet.

Nach einer anderweitigen vorteilhaften Ausgestaltung der Erfindung ist der optische Aufsatz bis zu dem der Bilderfassungseinrichtung gegenüberliegenden Rand des Tablet-PC's geführt. Dadurch erhält das Videozentriersystem ein gefälliges symmetrisches Erscheinungsbild.

Nach einer zusätzlichen vorteilhaften Ausgestaltung der Erfindung ist der optische Aufsatz lösbar mit dem Tablet-PC verbunden. Dadurch ist es möglich, unterschiedliche Aufsätze zu verwenden. Außerdem kann der Tablet-PC ohne optischen Aufsatz in seiner ursprünglichen Beschaffenheit bequemer für andere Zwecke als die der Videozentrierung gehandhabt werden.

Weitere Vorteile und vorteilhafte Ausgestaltungen der Erfindung sind der nachfolgenden Beschreibung, den Zeichnungen und den Ansprüchen entnehmbar.

### Zeichnung

Ein Ausführungsbeispiel der Erfindung ist in der Zeichnung dargestellt und im Folgenden näher beschrieben. In der Zeichnung zeigen:
- Fig. 1: eine räumliche Darstellung des mobilen Videozentriersystems und
- Fig. 2: den Grundaufbau des optischen Aufsatzes.

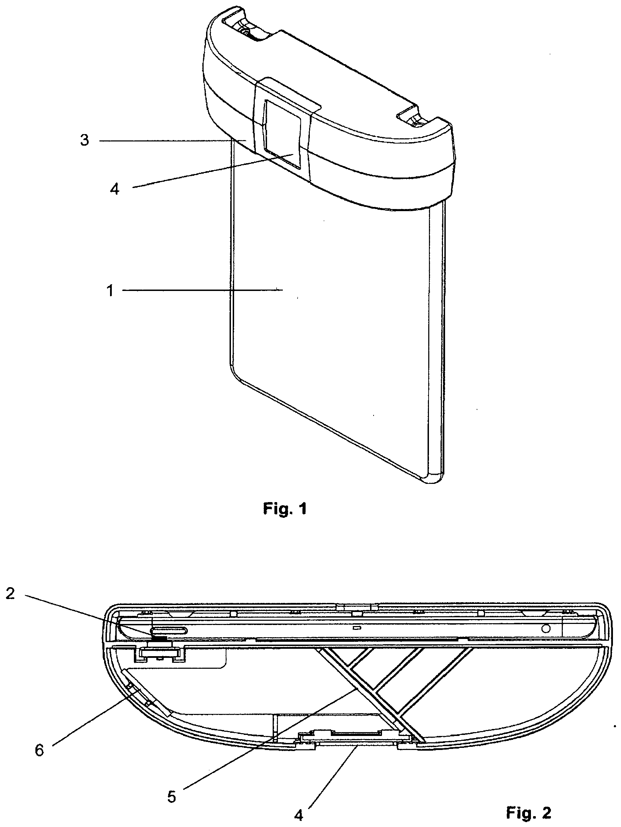

Wie aus den Fig. 1 und 2 zu erkennen, besteht das erfindungsgemäße mobile Videozentriersystem aus einem Tablet-PC 1 mit einem Display, einer auf der dem Display abgewandten Seite in einer vom Betrachter der Figuren aus gesehen rechten oberen Ecke befindlichen hochauflösenden Kamera 2, sowie einem eingebauten, nicht näher dargestellten Lagesensor, der dem Bediener auf dem Display Informationen zur Lage des Videozentriersystems im Raum gibt. Im vorliegenden Beispiel wurde ein Tablet-PC mit einem 7,9 Zoll-Display verwendet. Die hochauflösende Kamera 2 hat eine Auflösung von 8 Megapixel. Am oberen Rand des Tablet-PC's ist ein sich von seinem vertikalen linken Rand, dabei das Objektiv der hochauflösenden Kamera 2 überdeckend, bis zu seinem vertikalen rechten Rand erstreckender optischer Aufsatz 3 angeordnet. Im vorliegenden Beispiel ist der optische Aufsatz 3 lösbar mit dem Tablet-PC 1 verbunden. In der Mitte des optischen Aufsatzes 3 befindet sich eine Eintrittsöffnung 4, hinter der ein erster Umlenkspiegel 5 angeordnet ist. Im Verlauf des sich in Richtung der hochauflösenden Kamera 2 erstreckenden Strahlenganges ist ein zweiter Umlenkspiegel 6 angeordnet, wobei sich der zweite Umlenkspiegel 6 in der optischen Achse der hochauflösenden Kamera 2 befindet.

Nachfolgend wird die Handhabung des mobilen Videozentriersystems beschrieben. Die bedienende Person, in der Regel ein Optiker, hält den Tablet-PC 1 in Höhe der Augen eines Probanden, für dessen Brille die Zentrierdaten bestimmt werden sollen. Dabei bilden der Optiker, die vertikale Mitte des Tabelt-PC's 1 und die Mitte des Gesichts des Probanden eine Gerade, die sog. Fixierlinie. Mit Hilfe des in den Tablet-PC 1 eingebauten Lagesensors, der die Funktion einer elektronischen Wasserwaage ausübt, kann der Bediener die Achse der Eintrittsöffnung 4 genau auf die Blickachse des Probanden ausrichten, um eine Frontalaufnahme von dessen Gesicht zu machen. Die elektronische Vergrößerung der hochauflösenden Kamera 2 des Tablet-PC 1 wird so eingestellt, dass sich auch in einer größeren Entfernung des Optikers vom Probanden, d. h. in etwa 0,7 m Entfernung, das Gesicht des Probanden formatfüllend auf dem Suchbildschirm des Tablet-PC's 1 abbildet. Nach der Auslösung der hochauflösenden Kamera 2 ist das Bild gespeichert und kann zur Ermittlung der Zentrierdaten ausgewertet werden. Dadurch, dass der Tablet-PC 1 zur Aufnahme des Gesichts des Probanden in dessen Augenhöhe bewegt wird, braucht zur Ermittlung der Zentrierdaten kein Neigungswinkel gemessen und berücksichtigt zu werden, da die Aufnahmebedingung der Nullblickrichtung durch den Optiker eingehalten wird.

### Bezugszahlenliste

- 1: Tablet-PC
- 2: Hochauflösende Kamera
- 3: Optischer Aufsatz
- 4: Eintrittsöffnung
- 5: Erster Umlenkspiegel
- 6: Zweiter Umlenkspiegel

## Patentansprüche

1. Mobiles Videozentriersystem zur Bestimmung von Zentrierdaten für Brillengläser, bestehend aus einem Tablet-PC (1), der ein Display, einen Lagesensor und an einem der beiden vertikalen Randbereiche der dem Display abgewandten Seite eine Bilderfassungseinrichtung (2) aufweist, wobei diese Seite des Tablet-PC's (1) mit einem vom Objektiv dieser Bilderfassungseinrichtung (2) zumindest bis zur Mitte des Tablet-PC's (1) reichenden optischen Aufsatz (3) versehen ist, der eine Austrittsöffnung (4) aufweist, die sich in der Ebene der vertikalen Mitte dieser Seite des Tablet-PC's (1) befindet,
**dadurch gekennzeichnet,**
**dass** der Tablet-PC (1) eine hochauflösende Bilderfassungseinrichtung (2) aufweist und
**dass** in dem optischen Aufsatz (3) ein Periskop mit Umlenkspiegeln (5, 6) angeordnet ist.

2. Mobiles Videozentriersystem nach Anspruch 1,
**dadurch gekennzeichnet,**
**dass** das Display des Tablet-PC's (1) nicht größer als 8 Zoll ist.

3. Mobiles Videozentriersystem nach Anspruch 1 oder 2,
**dadurch gekennzeichnet,**
**dass** der optische Aufsatz (3) parallel zum oberen Rand des Tablet-PC's (1) angeordnet ist.

4. Mobiles Videozentriersystem nach Anspruch 1, 2 oder 3,
**dadurch gekennzeichnet,**
**dass** der optische Aufsatz (3) bis zu dem der hochauflösenden Bilderfassungseinrichtung (2) gegenüberliegenden Rand des Tablet-PC's (1) geführt ist.

5. Mobiles Videozentriersystem nach einem der Ansprüche 1 bis 4,
**dadurch gekennzeichnet,**
**dass** der optische Aufsatz (3) lösbar mit dem Tablet-PC (1) verbunden ist.
